# EUROPÄISCHE PATENTSCHRIFT

(11) **EP 0 760 361 B1**
(45) Veröffentlichungstag und Bekanntmachung des Hinweises auf die Patenterteilung: **10.01.2001**
(21) Anmeldenummer: 96113143.0
(22) Anmeldetag: 16.08.1996
(51) Int. Cl.: C07C 209/74, C07B 35/06, C07C 211/28

(54) **Verfahren zur racemisierungsfreien Dehalogenierung von halogensubstituierten Aryl-alkylaminen**
Process for the dehalogenation without racemisation of halogeno-substituted aryl-alkylamines
Procédé de déhalogénation sans racémisation d'arylalkylamines substituées par des halogènes

(30) Priorität: 24.08.1995 DE 19531116
(43) Veröffentlichungstag der Anmeldung: 05.03.1997
(73) Patentinhaber: BASF AKTIENGESELLSCHAFT, 67056 Ludwigshafen (DE)
(72) Erfinder: Ditrich, Klaus, Dr., 67161 Gönnheim (DE)

(56) Entgegenhaltungen:
- PATENT ABSTRACTS OF JAPAN vol. 010, no. 085 (C-336), 4.April 1986 & JP 60 218330 A (MITSUI TOATSU KAGAKU KK), 1.November 1985,
- PAUL RYLANDER: "Catalytic hydrogenation in organic syntheses" 1979 , ACADEMIC PRESS , NEW YORK - SAN FRANCISCO - LONDON XP002054886 * Seite 235 - Seite 242 *
- S.V. THIRUVIKRAMAN ET AL.: "S-4-Chlorotryptophan...." TETRAHEDRON LETTERS, Bd. 29, Nr. 19, 1988, OXFORD GB, Seiten 2339-2342, XP002019586

## Beschreibung

Die vorliegende Erfindung betrifft ein Verfahren zur racemisierungsfreien Dehalogenierung von halogensubstituierten Arylalkylaminen der Formel I, die ein optisches Zentrum an dem mit * gekennzeichneten C-Atom besitzen 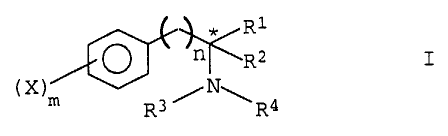 wobei
- m =: 1, 2, 3;
- n =: 0 - 4;
- R¹, R²,: unabhängig voneinander H, C₁-C₁₀-Alkyl, Aryl oder miteinander zusammen mit dem * C-Atom ein 3 bis 8-gliedrigen Ring bilden;
- R³, R⁴: unabhängig voneinander H, C₁-C₁₀-Alkyl, Aryl oder miteinander zusammen mit den N-Atom einen 3 bis 8-gliedrigen Ring bilden oder mit R¹ oder R² einen 3 bis 8-gliedrigen Ring bilden; und
- X =: Cl, Br, J,
bedeuten.

Die Dehalogenierung von Aromaten beispielsweise mittels Wasserstoff unter Verwendung eines Raney-Nickel Katalysators ist bekannt (R.L. Augustine, "Catalytic Hydrogenation", Marcel Dekker, New York, 1965, Seite 46 und 125).

J.B.A. Thijssen et al., Synthesis of ³H-labelled Levamisole, Journal of labelled Compounds and Radiopharmaceuticals 1987, 24, 7 779-786 beschreiben einen Brom-Tritium-Austausch an einem Arylalkyldiamin durch Tritiumgas und Pd/C unter Erhalt des optischen Zentrums.

JP 218 330/85 beschreibt ein Verfahren zur Dehalogenierung von Verbindungen, die einen Halogenrest und einen O- oder N-Benzylrest tragen unter Erhalt der Benzyl-Gruppe.

Weiterhin ist aus DE 28 51 039 bekannt, daß Arylalkylamine, die ein optisch aktives Zentrum (*) besitzen, unter den o.g. Hydrierbedingungen (Wasserstoff, Raney-Nickel Katalysator) racemisieren:

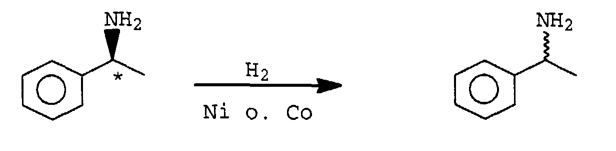

Es bestand die Aufgabe, ein Verfahren zur Dehalogenierung von Arylalkylaminen-Enantiomeren unter Erhalt der optischen Aktivität am die Aminogruppe tragenden C-Atom bereitzustellen.

Überraschenderweise wurde nun ein Verfahren zur Dehalogenierung von halogensubstituierten Arylalkylaminen gefunden, das dadurch gekennzeichnet ist, daß man Enantiomere der Formel I, die ein optisches Zentrum an dem mit * gekennzeichneten C-Atom besitzen 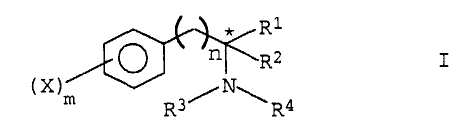 wobei
- m =: 1, 2, 3;
- n =: 0 - 4;
- R¹, R²: unabhängig voneinander H, C₁-C₁₀-Alkyl, Aryl oder miteinander zusammen mit dem * C-Atom ein 3 bis 8-gliedrigen Ring bilden;
- R³, R⁴: unabhängig voneinander H, C₁-C₁₀-Alkyl, Aryl oder miteinander zusammen mit dem N-Atom einen 3 bis 8-gliedrigen Ring bilden oder mit R¹ oder R² einen 3 bis 8-gliedrigen Ring bilden; und
- X =: Cl, Br, J,
bedeuten,
mit einem Hydrierungsmittel in Gegenwart eines Hydrierkatalysators, enthaltend Übergangsmetalle, ausgewählt aus der Gruppe Pd, Fe, Co, Ni zu Enantiomeren der Formel II 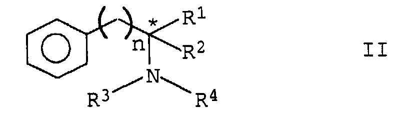 unter Erhalt des optischen Zentrums * umsetzt.

Als Hydrierungsmittel eignen sich Wasserstoff oder Salze der Ameisensäure wie Natriumformiat und Ammoniumformiat. Bevorzugt wird als Hydrierungsmittel gasförmiger Wasserstoff in einem Druckbereich zwischen Normaldruck und 500 bar, insbesondere zwischen Normaldruck und 100 bar, verwendet.

Als Katalysatoren eignen sich alle gebräuchlichen Hydrierkatalysatoren, vor allem solche, die Übergangsmetalle wie Ni, Co, Fe, Pd enthalten.

Die Katalysatoren können sowohl in homogener Phase, beispielsweise als Triphenylphosphinkomplexe als auch in immobilisierter Form eingesetzt werden.

Bevorzugt werden für das erfindungsgemäße Verfahren geträgerte Katalysatoren eingesetzt. Träger sind beispielsweise Siliziumdioxid, Aluminiumoxid, Bims, Kohle und andere dem Fachmann bekannte Träger.

Bevorzugt werden Raney-Katalysatoren, wie Raney-Nickel, Raney-Kobalt, Raney-Nickel-Eisen, Raney-Nickel-Kobalt oder Raney-Nickel-Eisen-Kobalt in wasserfreier oder auch wasserfeuchter oder lösungsmittelfeuchter Form eingesetzt.

Weiter bevorzugte Katalysatoren sind Palladium-haltige Katalysatoren, insbesondere Palladium auf Aktivkohle.

Die Katalysatoren werden in einer Menge von 1 - 25 Gew.-%, bevorzugt von 2,5 bis 12,5 Gew.-%, bezogen auf das zu dehalogenierende Substrat eingesetzt.

Die Dehalogenierung kann bei Temperaturen zwischen 0 und 250°C, bevorzugt zwischen 10 und 50°C ausgeführt werden.

Wenn mit Raney-Nickel als Katalysator gearbeitet wird empfiehlt es sich bei schonenden Bedingungen zu hydrieren, beispielsweise bei Raumtemperatur, damit nicht durch zu drastische Bedingungen eine Dehydrierung/Hydrierung am optischen Zentrum und damit eine unerwünschte Racemisierung eintritt.

Da bei der Dehalogenierung in stöchiometrischer Menge Halogenwasserstoff gebildet wird, empfiehlt sich die Zugabe einer Base, beispielsweise eines Amins oder eines Alkali- oder Erdalkalihydroxids zu der Reaktionslösung.

Es kann aber auch mit gutem Erfolg ohne Zugabe einer Base gearbeitet werden.

Als Lösungsmittel eignen sich alle gegen hydrierende Bedingungen beständige Lösungsmittel, beispielsweise Alkohole wie Methanol, Ethanol, Propanol, Butanol, Ether wie MTBE, THF, Dioxan, Carbonsäuren wie Eisessig, Propionsäure, Kohlenwasserstoffe wie Hexan, Cyclohexan oder auch Wasser, sowie Mischungen der genannten Lösungsmittel.

Das erfindungsgemäße Verfahren ist hinsichtlich der Substrate mit einer Vielzahl von Arylalkylaminen, die mit der allgemeinen Formel I beschrieben werden, durchführbar.

Bevorzugt werden mit dem erfindungsgemäßen Verfahren die in Patentanspruch 6 genannten Arylalkylamine dehalogeniert.

Die folgenden Beispiele dienen der weiteren Veranschaulichung der Erfindung.

### Beispiel 1

### Dehalogenierung mit Pd-Katalysator

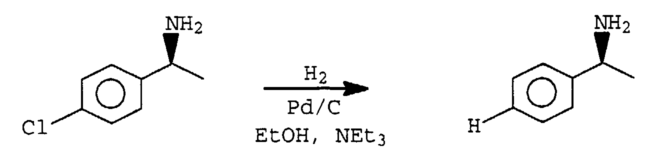

2,0 g (13 mMol) l-(4-Chlorphenyl)-ethylamin S-Enantiomer (ee=98% mit HPLC bestimmt) wurde in 10 ml Ethanol gelöst, mit 3,0 g Triethylamin versetzt und 100 mg Pd auf Aktivkohle zugegeben. Bei Raumtemperatur wurde im Autoklaven bei 20 bar Wasserstoffdruck 20 Stunden gerührt, anschließend der Katalysator über Kieselgur abfiltriert und das Filtrat auf ca. 5 ml eingeengt. Das Filtrat wurde mit 10 ml 20%iger NaOH verdünnt und mit Ether extrahiert. Die vereinigten Etherextrakte wurden vom Lösungsmittel befreit.

Als Rückstand blieb 1,3 g (81% d.Th.) (S)-1-Phenylethylamin als farbloses Öl mit ee=98% (mit HPLC bestimmt).

1H-NMR (CDCl₃:
δ = 1,30 (d, J = 7 Hz; 3H); 2,0 (s, breit, 2H); 4,1 (q, J = 7 Hz, 1H); 7,30 (m, 5H).

### Beispiel 2

### Dehalogenierung mit Pd-Katalysator ohne Base

5,0 g 1-(4-Chlorphenyl)-ethylamin S-Enantiomer (ee=96,7% mit HPLC bestimmt) wurde in 20 ml Ethanol gelöst, mit 100 mg Pd auf Aktivkohle versetzt und über Nacht bei Raumtemperatur und 40 bar Wasserstoffdruck gerührt. Anschließend wurde der Katalysator über Kieselgur abfiltriert und das Filtrat zur Trockenheit eingedampft. Der feste Rückstand wurde in 10 ml Wasser gelöst, mit NaOH-Lösung alkalisch gestellt, mit Ether extrahiert und die Extrakte getrocknet. Nach Entfernen des Extraktionsmittels erhielt man 3,4 g (87% d.Th.) (S)-Phenylethylamin (ee=96,7%).

### Beispiel 3

### Dehalogenierung mit Raney-Nickel Katalysator

4,0 g 1-(4-Chlorphenyl)-ethylamin S-Enantiomer (ee=97% mit HPLC bestimmt) wurde in 20 ml Ethanol gelöst, mit 6 ml Triethylamin versetzt und 100 mg Raney-Nickel zugegeben. Bei Raumtemperatur wurde über Nacht bei 40 bar Wasserstoffdruck gerührt. Am nächsten Tag wurde vom Katalysator abfiltriert und wie in Beispiel 1 beschrieben aufgearbeitet.
Ausbeute: 2,7 g (87% d.Th.) (S)-Phenylethylamin (ee=97%)

## Patentansprüche

1. Verfahren zur Dehalogenierung von halogensubstituierten Arylalkylaminen, dadurch gekennzeichnet, daß man Enantiomere der Formel I, die ein optisches Zentrum an dem mit ∗ gekennzeichneten C-Atom besitzen 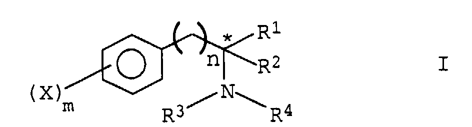 wobei
m = 1, 2, 3;
n = 0 - 4;
R¹, R² unabhängig voneinander H, C₁-C₁₀-Alkyl, Aryl oder miteinander zusammen mit dem * C-Atom ein 3 bis 8-gliedrigen Ring bilden;
R³, R⁴ unabhängig voneinander H, C₁-C₁₀-Alkyl, Aryl oder miteinander zusammen mit dem N-Atom einen 3 bis 8-gliedrigen Ring bilden oder mit R¹ oder R² einen 3 bis 8-gliedrigen Ring bilden; und
X = Cl, Br, J,
bedeuten,
mit einem Hydrierungsmittel in Gegenwart eines Hydrierkatalysators, enthaltend Übergangsmetalle, ausgewählt aus der Gruppe Pd, Fe, Co, Ni zu Enantiomeren der Formel II 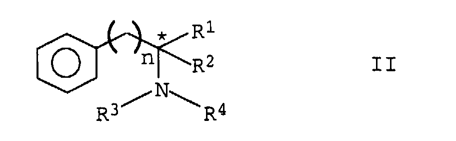 unter Erhalt des optischen Zentrums * umsetzt.

2. Verfahren nach Anspruch 1, dadurch gekennzeichnet, daß die Dehalogenierung in Gegenwart einer Base durchgeführt wird.

3. Verfahren nach Anspruch 1, dadurch gekennzeichnet, daß als Hydrierkatalysator ein Pd-haltiger Katalysator verwendet wird.

4. Verfahren nach Anspruch 3, dadurch gekennzeichnet, daß Pd auf Aktivkohle verwendet wird.

5. Verfahren nach Anspruch 1, dadurch gekennzeichnet, daß als Hydrierkatalysator Raney-Nickel verwendet wird.

6. Verfahren nach einem der Ansprüche 1-5, dadurch gekennzeichnet, daß in Formel I m =1; n=0; und R3, R4 = H bedeuten.

7. Verfahren nach Anspruch 5, dadurch gekennzeichnet, daß R1 = Methyl und R2 = H bedeuten.

## Claims

1. A process for dehalogenating halo-substituted arylalkyl-amines, which comprises reacting enantiomers of the formula I, which have an optical center at the C atom marked by * 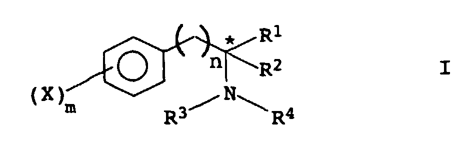 where
m = 1, 2 or 3;
n = 0 - 4;
R¹ and R² independently of one another are H, C₁-C₁₀-alkyl, aryl or with one another, together with the * C atom, form a 3 to 8-membered ring;
R³ and R⁴ independently of one another are H, C₁-C₁₀-alkyl, aryl or with one another, together with the N atom, form a 3 to 8-membered ring or, with R¹ or R², form a 3 to 8-membered ring; and
X = Cl, Br or I,
with a hydrogenating agent in the presence of a hydrogenation catalyst comprising transition metals selected from the group consisting of Pd, Fe, Co, Ni to give enantiomers of the formula II 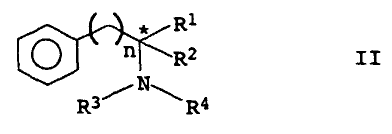 with retention of the optical center *.

2. A process as claimed in claim 1, wherein the dehalogenation is carried out in the presence of a base.

3. A process as claimed in claim 1, wherein the hydrogenation catalyst used is a Pd-containing catalyst.

4. A process as claimed in claim 3, wherein Pd on active carbon is used.

5. A process as claimed in claim 1, wherein the hydrogenation catalyst used is Raney nickel.

6. A process as claimed in one of claims 1-5, wherein in formula I m = 1, n = 0, and R³ and R⁴ = H.

7. A process as claimed in claim 5, wherein R¹ = methyl and R² = H.

## Revendications

1. Procédé pour déshalogéner des arylalkylamines portant des substituants halogéno, caractérisé par le fait que l'on fait réagir des énantiomères de formule I, présentant un centre optique sur l'atome de carbone signalé par l'astérisque 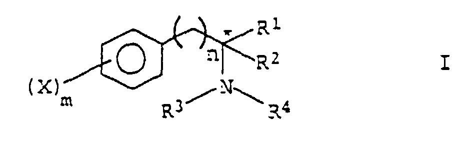 dans laquelle
m = 1, 2, 3 ;
n = 0 à 4 ;
R¹, R² : représentent chacun, indépendamment l'un de l'autre, H, un groupe alkyle en C1-C10, aryle,
ou forment ensemble et avec l'atome de carbone signalé par l'astérisque un cycle de trois à huit chaînons ;
R³, R⁴ : représentent chacun, indépendamment l'un de l'autre, H, un groupe alkyle en C1-C10, aryle,
ou forment ensemble et avec l'atome d'azote un cycle de trois à huit chaînons ou bien, avec R¹ ou R², un cycle de trois à huit chaînons ; et
X = Cl, Br, I,
avec un agent hydrogénant en présence d'un catalyseur d'hydrogénation contenant des métaux de transition choisis dans le groupe formé par Pd, Fe, Co, Ni, ce qui donne des énantiomères de formule II 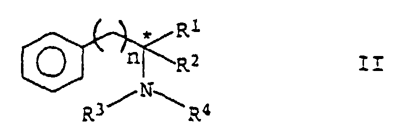 dans lesquels le centre optique * est conservé.

2. Procédé selon la revendication 1, caractérisé par le fait que la déshalogénation est réalisée en présence d'une base.

3. Procédé selon la revendication 1, caractérisé par le fait que l'on utilise en tant que catalyseur d'hydrogénation un catalyseur contenant Pd.

4. Procédé selon la revendication 1, caractérisé par le fait que l'on utilise Pd sur charbon actif.

5. Procédé selon la revendication 1, caractérisé par le fait que l'on utilise en tant que catalyseur d'hydrogénation le nickel de Raney.

6. Procédé selon l'une des revendications 1 à 5, caractérisé par le fait que, dans la formule I, m = 1 ; n = 0 ; et R³, R⁴ = H.

7. Procédé selon la revendication 5, caractérisé par le fait que R¹ = méthyle et R² = H.
